# EUROPEAN PATENT SPECIFICATION

(11) **EP 0 270 572 B1**
(45) Date of publication and mention of the grant of the patent: **20.10.1993**
(21) Application number: 87903201.9
(22) Date of filing: 04.05.1987
(51) Int. Cl.: A61M 5/165

(54) **FILTER MEANS FOR USE WITH SYRINGE AND NEEDLE**
FILTER ZUR VERWENDUNG MIT SPRITZE UND INJEKTIONSNADEL
ORGANE DE FILTRE DESTINE A ETRE UTILISE AVEC UNE SERINGUE ET UNE AIGUILLE

(30) Priority: 06.05.1986 US 860104
(43) Date of publication of application: 15.06.1988
(73) Proprietor: MICROGON, INC., Laguna Hills, CA 92653 (US)
(72) Inventor: CRONIN, James, J., Mission Viejo, CA 92692 (US)
(74) Representative: Baillie, Iain Cameron
(86) International application number: US8701041
(87) International publication number: WO8706845

(56) References cited:
- WO-A-85/00986
- GB-A- 2 153 247
- US-A- 4 014 797
- US-A- 4 066 079
- US-A- 4 137 917
- US-A- 4 267 053
- US-A- 4 391 274
- US-A- 4 453 927

## Description

### BACKGROUND OF THE INVENTION

This application is a Continuation-in-Part of co-pending application Serial No. 06/762,259, filed August 5, 1985.

This invention relates generally to a filtering device adapted to be used with a syringe and a needle; and, in another embodiment of the invention, to a combination syringe, needle and filter.

The current state of the art of syringe filters is believed to involve the use of a round flat sheet microporous membrane that is sealed between two plastic halves as an intermediate filter typically for use between a syringe and needle. If air is introduced to the syringe side of one of these filters and pressure is applied, the air is compressed over the entire surface area of the membrane thus substantially blocking or minimizing the flow of fluid. It will be appreciated, that if liquid is introduced to the syringe side of the flat filter (which is horizontal to the vertical axis of the syringe and needle), the liquid will only cover the entire surface of the membrane, in most cases, if the syringe and needle are in a vertical position. If the syringe and needle are in a horizontal position, or angled, the liquid may only cover part of the surface of the membrane thus causing air blockage. Moreover, increased filtering capacity of such filters can only be attained by increasing the diameter resulting in filters having dimensions that sometimes exceed the diameters of the syringe barrels to which they are attached and result in awkward handling.

In recent years, attempts to devise various syringe filters have resulted in that shown in U.S. Patent 4,316,462 which discloses an adapter which extends between the needle that is already in place on the syringe and a second needle. A flat membrane filter is used in this adapter.

Other art of note should be considered. U.S. patent number 4,267,053 discloses an intravenous device including a filter that is particularly well suited for filtering small particulate matter.

U.S. patent number 4,137,917 discloses a syringe and filter, the filter movable in a direction transverse to the major axis of the syringe thereby enabling (1) the expulsion of air from the syringe, (2) the drawing in of fluid into the syringe, and (3) the ejection of the fluid from the syringe.

U.S. patent number 4,453,927 discloses a filter for use with a syringe, the syringe being particularly useful for small volume infusion of neonates.

Finally, G.B. 2,153,247 introduces an intravenous device making use of a tubular filter system which can be used in a non-vertical position and primed in 15 seconds.

According to this invention, there is provided a filtering device adapted to be used with a syringe and a needle wherein longitudinally positive hollow filter fibres and a flow blocking material are arranged within the tubular body for directing flow through the material of the hollow fibres. The device according to the invention can be embodied either as a single filter element or as a combination syringe, filter and needle.

The foregoing arrangement produces the advantage that, even though the syringe and needle are tipped from vertical during use, the fluid will pass through the filter fibers tangentially to the longitudinal axis of the filter, thus eliminating air blockage. If air is present, any air blockage that will occur will be minimal compared to the flat membrane filters.

### SUMMARY OF THE INVENTION

According to this invention, there has been provided a filtering device adapted to be used with a syringe and needle including an elongate tubular body having a first mounting means on the end thereof adapted to be sealingly connected to the needle mounting on said syringe and a second mounting means on the opposite end thereof adapted to be sealingly connected to the cooperative syringe mounting means on said needle. Elongate microporous hollow filter fibres are arranged within said tubular body with said fibres being closed at one end thereof and open at the opposite thereof. A flow blocking material is arranged around said fibres blocking flow through said tubular body other than through the material of the hollow fibres.

In accordance with another aspect of the invention, there is provided a combination syringe, needle and filter utilizing a filter of the foregoing type between the syringe and the needle.

In summary, disclosed herein is a filtering device adapted to be used with a syringe and a needle comprising:
an elongate tubular body having a first mounting means on one end thereof adapted to be sealingly connected to the needle mounting on said syringe and a second mounting means on the opposite end thereof adapted to be sealingly connected to the cooperative syringe mounting means on said needle; said device being characterized by:
elongate microporous hollow filter fibres arranged with said tubular body, said fibres being closed at one end thereof and open at the opposite end thereof, said fibres enable the ejection of and drawing in of fluids through said syringe without movement of said filter fibres; and,
flow blocking material arranged around said fibres for blocking flow through said tubular body other than through the material of said hollow fibres, said flow blocking material being located in said tubular body adjacent the end of said tubular body having said second mounting means, wherein the effective filtering area of said fibres is about .25 cm² to about 10 cm².

It was an object of this invention to provide a filter for a syringe and needle whereby the fluid drawn into the syringe through the needle or ejected from the syringe through the needle was filtered by a filter means intermediate the syringe and needle.

A further object of this invention was to provide a filter of the foregoing type which would eliminate or minimize air blockage.

A still further object of this invention was to provide a filter for use with a syringe and needle that would be easy to manufacturer and convenient to use.

These and other objects of the invention will be evident from the following more detailed description of the invention.

### DESCRIPTION OF THE DRAWINGS

The invention will be more fully understood and described with reference to the drawings wherein:
Fig. 1 is a view in cross-section of a syringe, needle and filter according to the invention;
Fig. 2 is a cross-sectional view of the filter according to the invention;
Fig. 3 is a view in cross-section taken on lines 3-3 of Fig. 2;
Fig. 4 is a view similar to Figure 2 showing an alternate embodiment of the invention; and,
Fig. 5 is a view in cross-section taken on lines 5-5 of Figure 4.

### DETAILED DESCRIPTION OF THE INVENTION

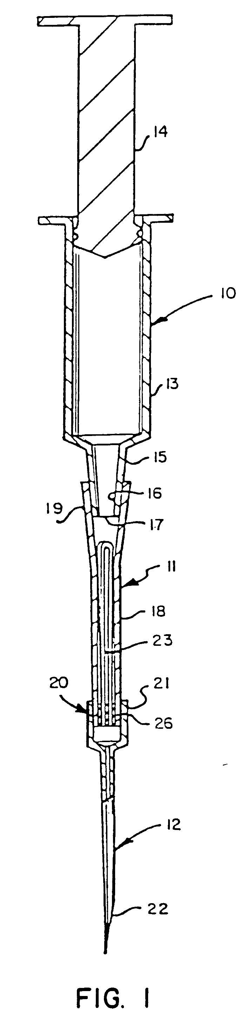

There is shown in Fig. 1 an arrangement according to the invention including a syringe 10, a filter 11 and a needle 12. The syringe has a barrel member 13 and a plunger element 14. The syringe includes a male needle mounting means 15 having a conical surface 16 that is normally adapted to mate with a mounting means on a needle such as needle 12. Conical surface 16 terminates in opening 17. Filter 11 includes an elongate tubular body 18 having a first mounting means 19 of outwardly tapered concial configuration adapted to mate with conical surface 16 of syringe 10 in a fluid sealing manner. At the opposite end of elongate tubular body 18 there is a second mounting means 20 of inwardly tapered conical configuration adapted to mate with corresponding mounting means of a needle such as needle 12.

Needle 12 includes an outwardly tapered female mounting means 21 of conical configuration and cannula 22.

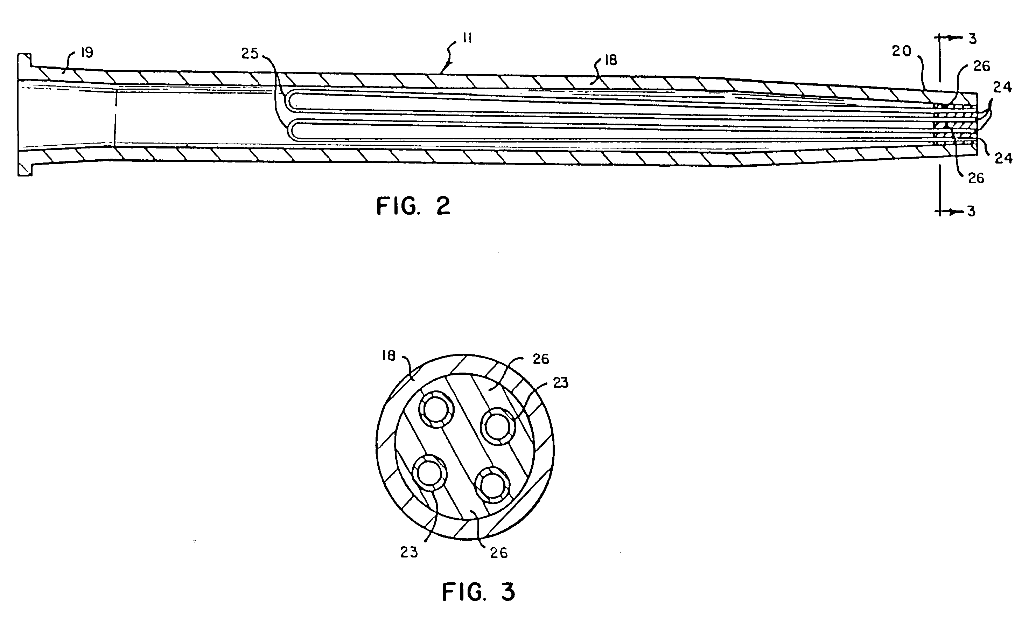

Mounted inside elongate tubular body 18 are elongate microporous hollow filter fibres 23. As best shown in Fig. 2, hollow fibres 23 have an open end 24 and a closed end 25. While a single fibre can be used having an open end and a sealed closed end, the arrangement shown in Fig. 2 wherein a fibre is looped back upon itself to form two open ends 24, with the loop providing closed ends 25, is preferred.

Flow blocking material 26 is arranged to position hollow filtering fibres 23 in elongate tubular body 18 such that flow through tubular body 18 other than through the material of hollow filtering fibres 23 is blocked. It is preferred to have the blocking material 26 in this position to provide a reservoir of fluid upstream of said material in said elongate tubular body 18 to promote transverse flow of said fluid material through the material of hollow filter fibres 23.

In a preferred form of the invention, the concial surfaces of elements 16, 19, 20 and 21 all have a conical angle of from about 1° to about 2°.

The elongate tubular body 18 of filter 11, in its preferred form, should not exceed the diameter of a barrel member 13 of syringe 10.

In a preferred form, the total length of filter unit 11 will be about 0.5 inches to about 4 inches (.197 cm to 1.57 cm) with a preferred outside diameter in the range of about .10 inches (.039 cm) to about .30 inches (.118 cm) with an inside diameter approximating about .05 inches (.019 cm) to about .20 inches (.079 cm). The ratio of length to the outside diameter of filter will be in the range of about 2 to about 20.

The microporous hollow fibres 23 can be of any length, preferably up to and including the length of tubular body 18 less the depth of protrustion of the male needle mounting means 15 which otherwise might damage the fibres. The fibres 23 can also be relatively short in length compared to the length of tubular body 18 provided that sufficient filtration surface is present for the particular application.

The porosity rating of fibres 23 is in the microporous range of about .05 to about 1 microns. A preferred range is about 0.1 to about 0.45 microns.

The inside diameter for fibres 23 can be from about .008 inches (.003 cm) to about 0.08 inches (.03 cm) with a preferred inside diameter being in the range of about .012 inches (.0047 cm) to about .05 inches (.019 cm) with a further preferred diameter being about .018 ± 10%. It has been found in accordance with the invention that larger fibres under the pressures applied by injection force on the syringe may collapse. Smaller diameter fibres also increase the packing density. The outside diameter should be that which will result in a wall thickness of about .001 inches (.00039 cm) to about .005 inches (.0019 cm) with a preferred wall thickness being about .0015 inches (.00059 cm) to about .004 inches (.0016 cm).

Flow blocking potting material 26 can be selected from the group consisting of silicone, polyurethane, epoxy, and cyanoacrylate ester resins with polyurethane being the currently preferred potting material. Elongate tubular body 18 in its preferred form is made out of a rigid plastic material, preferably transparent or transluscent, such as an acrylic or polycarbonate resin.

The effective filtration area of the microporous hollow fibres 23 in a given filter element 11 is in the range of about .25 cm² to about 10 cm² with a preferred range being about 5.0 cm² or less. The effective filtration area is measured on the inside walls of the fibres.

The porosity of fibres 23 should be greater than about 50% with a preferred range being about 65% to 85% with the upper range of porosity being limited at a point where the fibres have no structural integrity.

Packing density of fibres 23 in tubular body 18 as expressed in a ratio of cross-sectional areas of fibres to cross-sectional area of the lumen of tubular body 18 should be less than about 60%. The number of fibres 23 in tubular body 18 is preferably between 1 and 10 and more preferably between 2 and 8.

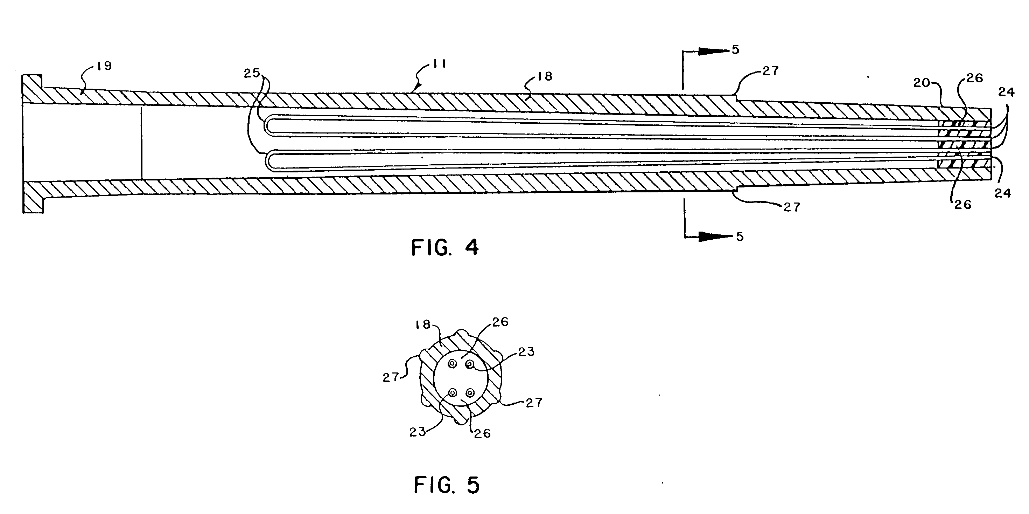

In Figure 4, there is shown an alternate embodiment according to the invention wherein the elongate tubular body is tapered, in this case, at an angle of about 1° to about 3° and wherein there are also included finger grippable projections in the form of an elongate ribs 27. These ribs extending substantially the length of the tubular portion 18 of filter unit 11. The existence of ribs 27 make it not only easier to grip the filter when it is connected with needle 12 and syringe 10, but further make it easier to install on the syringe and to facilitate removal and connection with needle 12.

An added advantage of the elongate filter 18 of the invention is that the filter itself will fit into ampoules for the withdrawal of liquids therefrom. The filter, therefore, has the advantage of filtering either in asperation or injection of fluids.

As best shown in Figure 5, grips 27 can be spaced equal distance around tubular body 18 and it is preferred to have such ribs projecting from said tubular body at a distance in the range of from .008 to .015 inches (.003 cm to .006 cm). The elongate ribs 27 are themselves tapered and it is preferred that the angle of taper be from about 1.0 to about 1.5 degrees. The taper of the tube 18 and the ribs 27 facilitate manufacture of the filter by the injection molding process whereby the parting line can be at the face 28 of a standard luer fitting 29. It is also preferred, to have the interior of tube 18 tapered at an angle substantially the same as that of the exterior to provide for easy removal from mold parts in the injection molding process.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description; and all changes which come within the meaning and range of equivalency of the claims are therefore intended to be embraced therein.

## Claims

1. A filtering device (11) adapted to be used with a syringe (10) and a needle (12) comprising:
an elongate tubular body (18) having a first mounting means on one end thereof adapted to be sealingly connected to the needle mounting on said syringe (10) and a second mounting means on the opposite end thereof adapted to be sealingly connected to the cooperative syringe mounting means on said needle (12); said device being characterized by:
elongate microporous hollow filter fibres (23) arranged with said tubular body (18), said fibres (23) being closed at one end thereof and open at the opposite end thereof, said fibres enable the ejection of and drawing in of fluids through said syringe (10) without movement of said filter fibres (23); and,
flow blocking material (26) arranged around said fibres (23) for blocking flow through said tubular body (18) other than through the material of said hollow fibres (23), said flow blocking material (26) being located in said tubular body (18) adjacent the end of said tubular body (18) having said second mounting means, wherein the effective filtering area of said fibres (23) is about .25 cm² to about 10 cm².

2. The filtering device (11) of claim 1 further characterized in that said first mounting means is adapted to be connected to a male needle mounting means of conical configuration of a syringe (10) and said second mounting means is adapted to be sealingly connected to a female mounting means of conical configuration of a needle (12).

3. The filtering device (11) of any one of the preceding claims further characterized in that said first and second mounting means are conical in configuration and adapted to mate with corresponding conical portions of said needle mounting means and said needle (12).

4. The filtering device (11) of claim 3 further characterized in that the conical angle of said conical mounting is about 1 degree to about 2 degrees.

5. The filtering device (11) of any one of the preceding claims further characterized in that the open end of said elongate microporous hollow fibres (23) is adjacent the end of said tubular body (18) having said second mounting means.

6. The filtering device (11) of any one of the preceding claims further characterized in that the ratio of length to the outside diameter of said elongate tubular body (18) is in the range of about 2 to 20.

7. The filtering device (11) of any one of the preceding claims further characterized in that the porosity rating of said fibres (23) is in the range of about 0.05 to about 1 micron.

8. The filtering device (11) of any one of the preceding claims further characterized in that the packing density of said fibres (23) is less than about 60%.

9. The filtering device (11) of any one of the preceding claims further characterized in that the porosity of said fibres (23) is greater than about 50%.

10. The filtering device (11) of any one of the preceding claims further characterized in that the elongated tubular body (18) is tapered.

11. The filtering device (11) of any one of the preceding claims further characterized in that the elongated tubular body (18) has finger grippable projections (27) extending outwardly therefrom.

12. The device of claim 11 wherein said finger grippable projections (27) comprise elongate ribs.

13. The device of claim 12 wherein said elongate ribs (27) are tapered.

14. A filtering device (11) adapted to be used with a syringe (10) or a syringe (10) and a needle (12), for filtering fluids from said syringe (10), said filtering device (11) comprising:
an elongate tubular body (18) having a first mounting means on one end thereof adapted to be sealingly connected to the needle mounting on said syringe (10) and a second mounting means on the opposite end thereof adapted to be sealingly connected to the cooperative syringe mounting means on said needle (12), said first mounting means being adapted to be connected to a male needle mounting means of conical configuration of a syringe (10) and said second mounting means being adapted to be sealingly connected to a female mounting means of conical configuration of a needle (12), said first and second mounting means being conical in configuration and adapted to mate with corresponding conical portions of said needle mounting means and said needle (12); said device being characterized by:
elongate microporous hollow filter fibres (23) arranged longitudinally within said tubular body (18), said fibres (23) being closed at one end thereof and open at the opposite end thereof, said filter fibres (23) enabling fluid to be drawn in or ejected through said syringe (10) without movement of said fibres (23);
flow blocking material (26) arranged around said fibres (23) for blocking flow through said tubular body (18) other than through the material of said hollow fibres (23), said flow blocking material (26) being provided at a position in said elongate tubular body 18 to provide with said elongate microporous hollow filter fibres (23) a reservoir of fluid around said hollow filter fibres (23) to promote transverse flow of said fluid through the material of said hollow filter fibers, the conical angle of said conical mounting being about 1 to 2 degrees, said flow blocking material (26) being located in said tubular body (18) adjacent the end of said tubular body (18) having said second mounting means, the open end of said elongate microporous hollow fibres (23) being adjacent the end of said tubular body (18) having said second mounting means, the ratio of length to the outside diameter of said elongate tubular body (18) being in the range of about 5 to 20, the porosity rating of said fibres (23) being in the range of about 0.05 to about 1 micron and the packing density of said fibres (23) being less than about 60%, the porosity of said fibres (23) being greater than about 50%, the effective filtering area of said fibres (23) being about 1 cm to about 10 cm, the total length of said filtering device (11) being about 0.5 inches (.1968 cm) to about 4 inches (1.574 cm), the outside diameter of said elongate tubular body (18) being about 0.10 inches (.039 cm) to about 0.30 inches (.118 cm) and the inside diameter of said elongate tubular body (18) being about 0.05 inches (.0197 cm) to about 0.20 inches (.079 cm).

15. A combination syringe (10), filter and needle (12) comprising:
a syringe (10) having a mounting means thereon;
a needle (12) having a mounting means thereon;
a filter for filtering fluids from said syringe (10), said filter comprising an elongate tubular body (18) having a first mounting means on one end thereof adapted to be sealingly connected to the needle mounting on said syringe 10 and a second mounting means on the opposite end thereof adapted to be sealingly connected to the cooperative syringe mounting means on said needle (12), said first mounting means being adapted to be connected to a male needle mounting means of conical configuration of a syringe (10) and said second mounting means being adapted to be sealingly connected to a female mounting means of conical configuration of a needle (12), said first and second mounting means being conical in configuration and adapted to mate with corresponding conical portions of said needle mounting means and said needle (12), said combination being characterized by:
elongate microporous hollow filter fibres (23) arranged longitudinally within said tubular body (18), said fibres (23) being closed at one end thereof and open at the opposite end thereof and enabling fluid to be drawn in or ejected from said syringe (10) without movement of said fibres (23);
flow blocking material (26) arranged around said fibres (23) for blocking flow through said tubular body (18) other than through the material of said hollow fibres (23), said flow blocking material (26) being provided at a position in said elongate tubular body (18) to provide with said elongate microporous hollow filter fibres (23) a reservoir of fluid around said hollow filter fibres (23) to promote transverse flow of said fluid through the material of said hollow filter fibers (23), the conical angle of said conical mounting being about 1 to 2 degrees, said flow blocking material (26) being located in said tubular body (18) adjacent the end of said tubular body (18) having said second mounting means, the open end of said elongate microporous hollow fibres (23) being adjacent the end of said tubular body (18) having said second mounting means, the ratio of length to the outside diameter of said elongate tubular body (18) being in the range of about 5 to 20, the porosity rating of said fibres (23) being in the range of about 0.05 to about 1 micron and the packing density of said fibres (23) being less than about 60%, the porosity of said fibres (23) being greater than about 50%, the effective filtering area of said fibres (23) being about 1 cm to about 10 cm the total length of said filtering device (11) being about 0.5 inches (.197 cm) to about 4 inches (1.574 cm), the outside diameter of said elongate tubular body (18) being about 0.10 inches (.039 cm) to about 0.30 inches (.118 cm) and the inside diameter of said elongate tubular body (18) being about 0.05 inches (.0197 cm) to about 0.20 inches (.079 cm).

## Patentansprüche

1. Filtervorrichtung (11), die in Verbindung mit einer Spritze (10) und einer Nadel (12) verwendet werden kann, umfassend:
einen langgestreckten rohrförmigen Körper (18) mit einer ersten Befestigungseinrichtung an seinem einen Ende, die dicht schließend mit der auf der Spritze (10) befestigten Nadel verbunden werden kann, und mit einer zweiten Befestigungseinrichtung an seinem entgegengesetzten Ende, die dicht schließend mit der zusammenwirkenden Spritzenbefestigungseinrichtung auf der Nadel (12) verbunden werden kann; wobei die Vorrichtung gekennzeichnet ist durch:
langgestreckte mikroporöse Filterfasern (23), die in dem rohrförmigen Körper (18) angeordnet sind, wobei die Fasern (23) an einem Ende geschlossen und an dem entgegengesetzten Ende offen sind, und wobei die Fasern das Ausstoßen und Einziehen von Flüssigkeiten durch die Spritze (10) ohne Bewegung der Filterfasern (23) ermöglichen; und
ein die Strömung hemmendes Material (26), das um die Fasern (23) herum angeordnet ist, um jede andere Strömung durch den rohrförmigen Körper (18) als die Strömung durch das Material der hohlen Fasern (23) zu hemmen, wobei das die Strömung hemmende Material (26) in dem rohrförmigen Körper (18) im Bereich des mit der zweiten Befestigungseinrichtung versehenen Endes des rohrförmigen Körpers (18) angeordnet ist, wobei die effektive Filterfläche der Fasern (23) etwa 0,25 cm² bis etwa 10 cm² beträgt.

2. Filtervorrichtung (11) nach Anspruch 1, ferner dadurch gekennzeichnet, daß die erste Befestigungseinrichtung mit einer außenkegelförmigen Nadelbefestigungseinrichtung einer Spritze (10) verbunden sein kann, und daß die zweite Befestigungseinrichtung dicht schließend mit einer innenkegelförmigen Befestigungseinrichtung einer Nadel (12) verbunden sein kann.

3. Filtervorrichtung (11) nach einem der vorhergehenden Ansprücher, ferner dadurch gekennzeichnet, daß die erste und die zweite Befestigungseinrichtung konisch geformt sind und mit entsprechenden konischen Abschnitten der Nadelbefestigungseinrichtung und der Nadel (12) zusammengreifen können.

4. Filtervorrichtung (11) nach Anspruch 3, ferner dadurch gekennzeichnet, daß der konische Winkel der konischen Befestigung etwa 1 Grad bis etwa 2 Grad beträgt.

5. Filtervorrichtung (11) nach einem der vorhergehenden Ansprüche, ferner dadurch gekennzeichnet, daß das offene Ende der langgestreckten mikroporösen Fasern (23) im Bereich des mit der zweiten Befestigungseinrichtung versehenen Endes des rohrförmigen Körpers (18) liegt.

6. Filtervorrichtung (11) nach einem der vorhergehenden Ansprüche, ferner dadurch gekennzeichnet, daß das Verhältnis der Länge zum Außendurchmesser des langgestreckten rohrförmigen Körpers (18) im Bereich von etwa 2 bis 20 liegt.

7. Filtervorrichtung (11) nach einem der vorhergehenden Ansprüche, ferner dadurch gekennzeichnet, daß der Porositätswert der Fasern (23) im Bereich von etwa 0,05 bis etwa 1 µm liegt.

8. Filtervorrichtung (11) nach einem der vorhergehenden Ansprüche, ferner dadurch gekennzeichnet, daß die Packungsdichte der Fasern (23) weniger als etwa 60% beträgt.

9. Filtervorrichtung (11) nach einem der vorhergehenden Ansprüche, ferner dadurch gekennzeichnet, daß die Porosität der Fasern (23) größer ist als etwa 50%.

10. Filtervorrichtung (11) nach einem der vorhergehenden Ansprüche, ferner dadurch gekennzeichnet, daß der langgestreckte rohrförmige Körper (18) konisch zuläuft.

11. Filtervorrichtung (11) nach einem der vorhergehenden Ansprüche, ferner dadurch gekennzeichnet, daß der langgestreckte rohrförmige Körper (18) mit den Fingern zu fassende Vorsprünge (27) aufweist, die von diesem nach außen ragen.

12. Vorrichtung nach Anspruch 11, dadurch gekennzeichnet, daß die mit den Fingern zu fassenden Vorsprünge (27) langgestreckte Rippen umfassen.

13. Vorrichtung nach Anspruch 12, dadurch gekennzeichnet, daß die langgestreckten Rippen (27) konisch zulaufen.

14. Filtervorrichtung (11), die in Verbindung mit einer Spritze (10) oder mit einer Spritze (10) und einer Nadel (12) verwendet werden kann, um Flüssigkeiten aus der Spritze (10) zu filtern, wobei die Filtervorrichtung (11) folgendes umfaßt:
einen langgestreckten rohrförmigen Körper (18) mit einer ersten Befestigungseinrichtung an seinem einen Ende, die dicht schließend mit der auf der Spritze (10) befestigten Nadel verbunden sein kann, und eine zweite Befestigungseinrichtung an seinem entgegengesetzten Ende, die dicht schließend mit der zusammenwirkenden Spritzenbefestigungseinrichtung auf der Nadel (12) verbunden sein kann, wobei die erste Befestigungseinrichtung mit einer außenkegelförmigen Nadelbefestigungseinrichtung einer Spritze (10) verbunden sein kann, und die zweite Befestigungseinrichtung dicht schließend mit einer innenkegelförmigen Befestigungseinrichtung einer Nadel (12) verbunden sein kann, wobei die erste und die zweite Befestigungseinrichtung konisch geformt sind und mit entsprechenden konischen Abschnitten der Nadelbefestigungseinrichtung und der Nadel (12) zusammengreifen können; wobei die Vorrichtung gekennzeichnet ist durch:
langgestreckte mikroporöse hohle Filterfasern (23), die in Längsrichtung im Innern des rohrförmigen Körpers (18) angeordnet sind, wobei die Fasern (23) an einem Ende geschlossen und am entgegengesetzten Ende offen sind, und wobei die Filterfasern (23) das Einziehen und Ausstoßen von Flüssigkeit durch die Spritze (10) ohne Bewegung der Fasern (23) ermöglichen;
ein die Strömung hemmendes Material (26), das um die Fasern (23) herum angeordnet ist, um jede andere Strömung durch den rohrförmigen Körper (18) als die Strömung durch das Material der hohlen Fasern (23) zu hemmen, wobei das die Strömung hemmende Material (26) an einer Position in dem langgestreckten rohrförmigen Körper (18) vorgesehen ist, die in den langgestreckten mikroporösen hohlen Filterfasern (23) einen Flüssigkeitsspeicher um die hohlen Filterfasern (23) bildet, um die quergerichtete Strömung der Flüssigkeit durch das Material der hohlen Filterfasern zu unterstützen, wobei der konische Winkel der konischen Befestigung etwa 1 bis 2 Grad beträgt, das die Strömung hemmende Material (26) im Innern des rohrförmigen Körpers (18) im Bereich des mit der zweiten Befestigungseinrichtung versehenen Endes des rohrförmigen Körpers (18) angeordnet ist, das offene Ende der langgestreckten mikroporösen hohlen Fasern (23) im Bereich des mit der zweiten Befestigungseinrichtung versehenen Endes des rohrförmigen Körpers (18) angeordnet ist, das Verhältnis der Länge zum Außendurchmesser des langgestreckten rohrförmigen Körpers (18) im Bereich von etwa 5 bis etwa 20 liegt, der Porositätswert der Fasern (23) im Bereich von etwa 0,05 bis etwa 1 µm liegt, und die Packungsdichte der Fasern (23) weniger als etwa 60% beträgt, die Porosität der Fasern (23) größer ist als etwa 50%, die effektive Filterfläche der Fasern (23) etwa 1 cm² bis etwa 10 cm² beträgt, die Gesamtlänge der Filtervorrichtung (11) etwa 0,1968 cm (0,5 Inch) bis etwa 1,574 cm (4 Inch) beträgt, der Außendurchmesser des langgestreckten rohrförmigen Körpers (18) etwa 0,039 cm (0,10 Inch) bis etwa 0,118 cm (0,30 Inch), und der Innendurchmesser des langgestreckten rohrförmigen Körpers (18) etwa 0,0197 cm (0,05 Inch) bis etwa 0,079 cm (0,20 Inch) beträgt.

15. Kombination aus Spritze (10), Filter und Nadel (12), umfassend:
eine Spritze (10) mit einer daran befindlichen Befestigungseinrichtung;
eine Nadel (12) mit einer daran befindlichen Befestigungseinrichtung;
einen Filter zum Filtern von Flüssigkeiten aus der Spritze (10), wobei der Filter einen langgestreckten rohrförmigen Körper (18) aufweist, der an seinem einen Ende eine erste Befestigungseinrichtung aufweist, die dicht schließend mit der auf der Spritze (10) befestigten Nadel verbunden sein kann, und eine zweite Befestigungseinrichtung an seinem entgegengesetzten Ende, die dicht schließend mit der zusammenwirkenden Spritzenbefestigungseinrichtung auf der Nadel (12) verbunden sein kann, wobei die erste Befestigungseinrichtung mit einer außenkegelförmigen Nadelbefestigungseinrichtung einer Spritze (10) verbunden sein kann, und die zweite Befestigungseinrichtung dicht schließend mit einer innenkegelförmigen Befestigungseinrichtung einer Nadel (12) verbunden sein kann, wobei die erste und die zweite Befestigungseinrichtung konisch geformt sind und mit entsprechenden konischen Abschnitten der Nadelbefestigungseinrichtung und der Nadel (12) zusammengreifen können, wobei die Kombination gekennzeichnet ist durch:
langgestreckte mikroporöse hohle Filterfasern (23), die in Längsrichtung im Innern des rohrförmigen Körpers (18) angeordnet sind, wobei die Fasern (23) an ihrem einen Ende geschlossen und an ihrem entgegengesetzten Ende offen sind und das Einziehen und Ausstoßen von Flüssigkeit in die bzw. aus der Spritze (10) ohne Bewegung der Fasern (23) ermöglichen;
ein die Strömung hemmendes Material (26), das um die Fasern (23) herum angeordnet ist, um jede andere Strömung durch den rohrförmigen Körper (18) als die Strömung durch das Material der hohlen Fasern (23) zu hemmen, wobei das die Strömung hemmende Material (26) an einer Position im Innern des langgestreckten rohrförmigen Körpers (18) vorgesehen ist, die in den langgestreckten mikroporösen hohlen Filterfasern (23) einen Flüssigkeitsspeicher um die hohlen Filterfasern (23) bildet, der die quergerichtete Strömung der Flüssigkeit durch das Material der hohlen Filterfasern (23) unterstützt, wobei der konische Winkel der konischen Befestigung etwa 1 bis 2 Grad beträgt, das die Strömung hemmende Material (26) im Innern des rohrförmigen Körpers (18) im Bereich des mit der zweiten Befestigungseinrichtung versehenen Endes des rohrförmigen Körpers (18) angeordnet ist, das offene Ende der langgestreckten mikroporösen hohlen Fasern (23) im Bereich des mit der zweiten Befestigungseinrichtung versehenen Endes des rohrförmigen Körpers (18) angeordnet ist, das Verhältnis der Länge zum Außendurchmesser des langgestreckten rohrförmigen Körpers (18) im Bereich von etwa 5 bis 20 liegt, der Porositätswert der Fasern (23) im Bereich von etwa 0,05 bis etwa 1 µm liegt, und die Packungsdichte der Fasern (23) weniger als etwa 60% beträgt, die Porosität der Fasern (23) größer ist als etwa 50%, die effektive Filterfläche der Fasern (23) etwa 1 cm² bis etwa 10 cm² beträgt, die Gesamtlänge der Filtervorrichtung (11) etwa 0,197 cm (0,5 Inch) bis etwa 1,574 cm (4 Inch) beträgt, der Außendurchmesser des langgestreckten rohrförmigen Körpers (18) etwa 0,039 cm (0,10 Inch) bis etwa 0,118 cm (0,30 Inch) beträgt, und der Innendurchmesser des langgestreckten rohrförmigen Körpers (18) etwa 0,0197 cm (0,05 Inch) bis etwa 0,079 cm (0,20 Inch) beträgt.

## Revendications

1. Dispositif de filtration (11) conçu pour être utilisé avec une seringue (10) et une aiguille (12), comprenant :
un corps tubulaire allongé (18) comportant un premier dispositif de montage à une extrémité, conçu pour être relié de manière étanche au dispositif de montage de l'aiguille sur ladite seringue (10) et un second dispositif de montage à l'extrémité opposée, conçu pour être relié de manière étanche au dispositif de montage de seringue qui coopère sur ladite aiguille (12), ledit dispositif étant caractérisé par :
des fibres filtrantes creuses microporeuses allongées (23) disposées dans ledit corps tubulaire (18), lesdites fibres (23) étant fermées à une extrémité et ouvertes à l'extrémité opposée, lesdites fibres permettant l'éjection de fluides de ladite seringue (10) et l'aspiration de fluides dans ladite seringue (10) sans mouvement desdites fibres filtrantes (23); et
une matière de blocage d'écoulement (26) disposée autour desdites fibres (23) pour bloquer l'écoulement dans ledit corps tubulaire (18) autrement qu'à travers la matière desdites fibres creuses (23), ladite matière de blocage d'écoulement (26) étant située dans ledit corps tubulaire (18) en position adjacente à l'extrémité dudit corps tubulaire (18) qui comporte ledit second dispositif de montage, la surface filtrante efficace desdites fibres (23) étant d'environ 0,25 cm² à environ 10 cm².

2. Dispositif de filtration (11) selon la revendication 1, caractérisé en outre en ce que ledit premier dispositif de montage est conçu pour être relié à un dispositif mâle de montage d'aiguille de configuration conique d'une seringue (10) et ledit second dispositif de montage est conçu pour être relié de manière étanche à un dispositif de montage femelle de configuration conique d'une aiguille (12).

3. Dispositif de filtration (11) selon l'une quelconque des revendications précédentes, caractérisé en outre en ce que lesdits premier et second dispositifs de montage sont de configuration conique et sont conçus pour s'adapter à des parties coniques correspondantes dudit dispositif de montage d'aiguille et de ladite aiguille (12).

4. Dispositif de filtration (11) selon la revendication 3, caractérisé en outre en ce que l'angle conique dudit dispositif de montage conique est d'environ 1° à environ 2°.

5. Dispositif de filtration (11) selon l'une quelconque des revendications précédentes, caractérisé en outre en ce que l'extrémité ouverte desdites fibres creuses microporeuses allongées (23) est adjacente à l'extrémité dudit corps tubulaire (18) qui comporte ledit second dispositif de montage.

6. Dispositif de filtration (11) selon l'une quelconque des revendications précédentes, caractérisé en outre en ce que le rapport de la longueur au diamètre externe dudit corps tubulaire allongé (18) est compris dans la plage d'environ 2 à 20.

7. Dispositif de filtration (11) selon l'une quelconque des revendications précédentes, caractérisé en outre en ce que la taille des pores desdites fibres (23) est comprise dans la plage d'environ 0,05 à environ 1 µm.

8. Dispositif de filtration (11) selon l'une quelconque des revendications précédentes, caractérisé en outre en ce que la densité de tassement desdites fibres (23) est inférieure à environ 60 %.

9. Dispositif de filtration (11) selon l'une quelconque des revendications précédentes, caractérisé en outre en ce que la porosité desdites fibres (23) est supérieure à environ 50 %.

10. Dispositif de filtration (11) selon l'une quelconque des revendications précédentes, caractérisé en outre en ce que le corps tubulaire allongé (18) est aminci.

11. Dispositif de filtration (11) selon l'une quelconque des revendications précédentes, caractérisé en outre en ce que le corps tubulaire allongé (18) comporte des protubérances (27) qui peuvent être saisies par les doigts et qui s'étendent à l'extérieur du corps.

12. Dispositif selon la revendication 11, dans lequel lesdites protubérances (27) qui peuvent être saisies par les doigts comprennent des nervures allongées.

13. Dispositif selon la revendication 12, dans lequel lesdites nervures allongées (27) sont amincies.

14. Dispositif de filtration (11) conçu pour être utilisé avec une seringue (10) ou avec une seringue (10) et une aiguille (12), pour filtrer les fluides provenant de ladite seringue (10), ledit dispositif de filtration (11) comprenant :
un corps tubulaire allongé (18) comportant un premier dispositif de montage à une extrémité, conçu pour être relié de manière étanche au dispositif de montage d'aiguille sur ladite seringue (10) et un second dispositif de montage à l'extrémité opposée, conçu pour être relié de manière étanche au dispositif de montage de seringue coopérant sur ladite aiguille (12), ledit premier dispositif de montage étant conçu pour être relié à un dispositif mâle de montage d'aiguille de configuration conique d'une seringue (10) et ledit second dispositif de montage étant conçu pour être relié de manière étanche à un dispositif de montage femelle de configuration conique d'une aiguille (12), lesdits premier et second dispositifs de montage étant de configuration conique et étant conçus pour s'adapter avec des parties coniques correspondantes dudit dispositif de montage d'aiguille et de ladite aiguille (12), ledit dispositif étant caractérisé par :
des fibres filtrantes creuses microporeuses allongées (23) disposées longitudinalement à l'intérieur dudit corps tubulaire (18), lesdites fibres (23) étant fermées à une extrémité et ouvertes à l'extrémité opposée, lesdites fibres filtrantes (23) permettant à un fluide d'être aspiré dans ladite seringue (10) ou d'être éjecté de ladite seringue (10) sans mouvement desdites fibres (23);
une matière de blocage d'écoulement (26) disposée autour desdites fibres (23) pour bloquer l'écoulement dans ledit corps tubulaire (18) autrement qu'à travers la matière desdites fibres creuses (23), ladite matière de blocage d'écoulement (26) étant placée en une position à l'intérieur dudit corps tubulaire allongé (18) de manière à former avec lesdites fibres filtrantes creuses microporeuses allongées (23) un réservoir de fluide autour desdites fibres filtrantes creuses (23) pour favoriser l'écoulement transversal dudit fluide à travers la matière desdites fibres filtrantes creuses, l'angle conique dudit dispositif de montage conique étant d'environ 1 à 2°, ladite matière de blocage d'écoulement (26) étant située dans ledit corps tubulaire (18) en position adjacente à l'extrémité dudit corps tubulaire (18) qui comporte ledit second dispositif de montage, l'extrémité ouverte desdites fibres creuses microporeuses allongées (23) étant adjacente à l'extrémité dudit corps tubulaire (18) qui comporte ledit second dispositif de montage, le rapport de la longueur au diamètre externe dudit corps tubulaire allongé (18) étant situé dans la plage d'environ 5 à 20, la taille des pores desdites fibres (23) étant située dans la plage d'environ 0,05 à environ 1 µm et la densité de tassement desdites fibres (23) étant inférieure à environ 60 %, la porosité desdites fibres (23) étant supérieure à environ 50 %, la surface filtrante efficace desdites fibres (23) étant d'environ 1 cm² à environ 10cm², la longueur totale dudit dispositif de filtration (11) étant d'environ 0,5 pouce (0,1968 cm) à environ 4 pouces (1,574 cm), le diamètre externe dudit corps tubulaire allongé (18) étant d'environ 0,10 pouce (0,039 cm) à environ 0,30 pouce (0,118 cm) et le diamètre interne dudit corps tubulaire allongé (18) étant d'environ 0,05 pouce (0,0197 cm) à environ 0,20 pouce (0,079 cm).

15. Combinaison d'une seringue (10), d'un filtre et d'une aiguille (12), comprenant :
une seringue (10) comportant un dispositif de montage :
une aiguille (12) comportant un dispositif de montage :
un filtre pour filtrer les fluides provenant de ladite seringue (10), ledit filtre comprenant un corps tubulaire allongé (18) comportant un premier dispositif de montage à une extrémité, conçu pour être relié de manière étanche au dispositif de montage d'aiguille sur ladite seringue (10) et un second dispositif de montage à l'extrémité opposée, conçu pour être relié de manière étanche au dispositif de montage de seringue coopérant sur ladite aiguille (12), ledit premier dispositif de montage étant conçu pour être relié à un dispositif mâle de montage d'aiguille de configuration conique d'une seringue (10) et ledit second dispositif de montage étant conçu pour être relié de manière étanche à un dispositif de montage femelle de configuration conique d'une aiguille (12), lesdits premier et second dispositifs de montage étant de configuration conique et étant conçus pour s'adapter avec des parties coniques correspondantes dudit dispositif de montage d'aiguille et de ladite aiguille (12), ladite combinaison étant caractérisée par :
des fibres filtrantes creuses microporeuses allongées (23) disposées longitudinalement à l'intérieur dudit corps tubulaire (18), lesdites fibres (23) étant fermées à une extrémité et ouvertes à l'extrémité opposée et permettant à un fluide d'être aspiré dans ladite seringue (10) ou d'être éjecté de ladite seringue (10) sans mouvement desdites fibres (23);
une matière de blocage d'écoulement (26) disposée autour desdites fibres (23) pour bloquer l'écoulement dans ledit corps tubulaire (18) autrement qu'à travers la matière desdites fibres creuses (23), ladite matière de blocage d'écoulement (26) étant placée en une position à l'intérieur dudit corps tubulaire allongé (18) de manière a former avec lesdites fibres filtrantes creuses microporeuses allongées (23) un réservoir de fluide autour desdites fibres filtrantes creuses (23) pour favoriser l'écoulement transversal dudit fluide à travers la matière desdites fibres filtrantes creuses, l'angle conique dudit dispositif de montage conique étant d'environ 1 à 2°, ladite matière de blocage d'écoulement (26) étant située dans ledit corps tubulaire (18) en position adjacente à l'extrémité dudit corps tubulaire (18) qui comporte ledit second dispositif de montage, l'extrémité ouverte desdites fibres creuses microporeuses allongées (23) étant adjacente à l'extrémité dudit corps tubulaire (18) qui comporte ledit second dispositif de montage, le rapport de la longueur au diamètre externe dudit corps tubulaire allongé (18) étant situé dans la plage d'environ 5 à 20, la taille des pores desdites fibres (23) étant située dans la plage d'environ 0,05 à environ 1 µm et la densité de tassement desdites fibres (23) étant inférieure à environ 60%, la porosité desdites fibres (23) étant supérieure à environ 50%, la surface filtrante efficace desdites fibres (23) étant d'environ 1 cm² à environ 10 cm², la longueur totale dudit dispositif de filtration (11) étant d'environ 0,5 pouce (0,1968 cm) à environ 4 pouces (1,574 cm), le diamètre externe dudit corps tubulaire allongé (18) étant d'environ 0,10 pouce (0,039 cm) à environ 0,30 pouce (0,118 cm) et le diamètre interne dudit corps tubulaire allongé (18) étant d'environ 0,05 pouce (0,0197 cm) à environ 0,20 pouce (0,079 cm).
